# EUROPÄISCHE PATENTSCHRIFT

(11) **EP 3 534 809 B1**
(45) Veröffentlichungstag und Bekanntmachung des Hinweises auf die Patenterteilung: **02.09.2020**
(21) Anmeldenummer: 17791668.1
(22) Anmeldetag: 27.10.2017
(51) Int. Cl.: A61B 17/32, A61B 17/54, A61N 7/00, A61H 33/00

(54) **DÉBRIDEMENT-VORRICHTUNG**
DEBRIDEMENT DEVICE
DISPOSITIF DE DÉBRIDEMENT

(30) Priorität: 02.11.2016 DE 102016221494
(43) Veröffentlichungstag der Anmeldung: 11.09.2019
(73) Patentinhaber: BSN Medical GmbH, 20253 Hamburg (DE)
(72) Erfinder: SCHÜTZ, Patrick, 12159 Berlin (DE); SCHRAMM, Torsten Matthias, 21073 Hamburg (DE)
(74) Vertreter: FARAGO Patentanwälte
(86) Internationale Anmeldenummer: PCT/EP2017/077539
(87) Internationale Veröffentlichungsnummer: WO 2018/083012

(56) Entgegenhaltungen:
- WO-A1-2016/116623
- WO-A1-2016/166347
- WO-A2-2008/040020
- WO-A2-2009/152374
- WO-A2-2011/121482
- US-A1- 2003 171 675

## Beschreibung

### Gegenstand der Erfindung

Die vorliegende Erfindung betrifft eine Ultraschallvorrichtung zur Abgabe einer Stickstoffmonoxid (NO)-haltigen Spüllösung zur Verwendung bei dem Debridement von Wunden.

### Hintergrund der Erfindung

Als Debridement oder Wundtoilette bezeichnet man das medizinische Vorgehen zur Entfernung von infiziertem, geschädigtem oder abgestorbenem (nekrotischem) Gewebe aus Geschwüren, Verbrennungs- und anderen Wunden oder bei Organzerfall (z. B. nekrotisierende Pankreatitis). Ziel ist es, die Wundbeobachtung zu gewährleisten, die Heilung zu initiieren, eine sekundäre Infektion des verbliebenen gesunden Gewebes zu verhindern und auf diese Weise ein Fortschreiten der Infektion, die zur Sepsis und Tod führen kann, zu stoppen. Zwar kann der Körper Nekrosen selbstständig abbauen, doch gerade bei großflächig verteilten Gewebetrümmern (z. B.: Verbrennung) besteht die Gefahr einer Intoxikation (Sepsis) mit irreversiblen Leberschäden. Es wurde inzwischen erkannt, dass ein effizientes Debridement eine Grundvoraussetzung für eine Funktionelle Gewebereparatur ist und es somit als medizinische Maßnahme von zentraler Bedeutung für die Versorgung schlecht heilender akuter und chronischer Wunden ist (Wundmanagement 2013, Suppl. 3, EWMA Dokument 2013: "Debridement").

Als effizientes Verfahren hat sich hier das Ultraschall-Debridement etabliert, das auch als Ultraschall-assistierte Wundreinigung bezeichnet wird. Die Wunde wird mit einer Spülflüssigkeit gespült und mit Ultraschall beschallt. Der Ultraschallimpuls treibt die eingeleitete Spülflüssigkeit bis in die tieferen Regionen der Wunde und löst Beläge und lose haftende Nekrosen.

Aus dem Stand der Technik sind zahlreiche Verfahren und Vorrichtungen zum Ultraschall-Debridement von Wunden bekannt.

Gemäß der WO 2004/014284 A1 kann eine Ultraschallspitze mit einem abrasiven Randbereich ausgestattet werden, um hier das Debridement der Wunde zu unterstützen oder das nekrotische Gewebe zu entfernen.

Die US 7,875,278 B2 lehrt einen Apparat zum Ultraschall-Debridement, bei dem der Ultraschallkopf als Löffel ausgestaltet ist, der randseitig mit Spitzen (502) für ein aggressives Debridement ausgestattet sein kann (s. Fig. 5).

Die WO 2008/040020 A2 betrifft eine Vorrichtung und ein Verfahren zur Behandlung von Wunden, Kavitäten und Knochen. Sie lehrt die Verwendung von gasförmigem NO ("nitric oxide gas") und somit ist das NO-Gas nicht als Bestandteil der Spül-Lösung oder als Bestandteil der eigentlichen Vorrichtung.

Die WO 2009/152374 A2 betrifft ein Verfahren zur Behandlung chronischer Wunden und lehrt die Verwendung einer Ultraschall-Hydrodebridement-Vorrichtung. Die WO'374 offenbart im Absatz [0027] lediglich die Verwendung von Stickstoff oder Sauerstoff als Gase und schweigt sich somit über die Verwendung von NO aus.

Es besteht daher noch Bedarf an neuen Vorrichtungen zum Ultraschall-Debridement, die die Ultraschallbehandlung verbessern und ein effektives Debridement auch bei schwer zu behandelnden Wunden erlauben.

Aufgabe der Erfindung ist es daher, eine Vorrichtung zum Ultraschall-Debridement bereitzustellen, die bezüglich mindestens einer der oben genannten Nachteile verbessert ist.

### Zusammenfassung der Erfindung

Diese Aufgabe wird gemäß der Erfindung dadurch gelöst, dass eine Ultraschallvorrichtung zur Abgabe einer Stickstoffmonoxid (NO)-haltigen Spüllösung bereitgestellt wird, die Folgendes umfasst:
(a) Ein Reaktionsgefäß zur Herstellung der NO-haltigen Spüllösung;
(b) Eine Duschvorrichtung mit einem Duschkopf zur Abgabe der NO-haltigen Spüllösung; und/oder
(c) Ein Badegefäß, das zur Aufnahme des zu behandelnden Körperteils und der NO-haltigen Spüllösung als Badelösung vorgesehen ist;
wobei der Duschkopf und/oder das Badegefäß mindestens einen Ultraschallwandler zur Ultraschallanregung der NO-haltigen Spül- oder Badelösung (und zur Ultraschallanregung tiefer Wundbereiche der damit behandelten Wunden) umfassen.

Spezifische Ausgestaltungen der Erfindung sind Gegenstand weiterer abhängiger Ansprüche.

Das beispielhafte Verfahren vereinigt mehrere entscheidende Vorteile gegenüber den aus dem Stand der Technik bekannten Verfahren.

Die Kombination einer Ultraschallbehandlung mit der Verwendung einer NO-haltigen Lösung hat sich im Rahmen der Debridementbehandlung als besonders vorteilhaft herausgestellt. Hierbei wirken die Ultraschallbehandlung und die NO-Behandlung in synergistischer Weise zusammen.

Die Ultraschallbehandlung kann einerseits eine verstärkte Koagulation hervorrufen. Durch die übertragene mechanische Energie kann auch bei geringen Mengen an Spüllösung ein wirksames Debridement erzielt werden. Durch die Fähigkeit hierbei auch tiefer gelegenes Wundgewebe zu erreichen und freizulegen, kann das Stickstoffmonoxid als bioaktive Substanz weitaus besser an die kritischen Zell- und Gewebestrukturen gelangen. Schädliche Mikroorganismen können durch das NO direkt *in situ* abgetötet werden und somit die Gefahr einer Verschleppung in andere Wund- oder Körperbereiche oder eine Ausbreitung in die Umgebung verringert werden.

Die Vorrichtung kann durch Einstellung der Ultraschallparameter und der Spülparameter (Wasserdruck, Temperatur oder Konzentration an NO) flexibel an die jeweilige Debridement-Situation angepasst werden.

Es gibt zahlreiche Ausgestaltungen für die Duschvorrichtung (verschiedene Düsenformen, Reinigungsaufsatz, Reinigungsmatrices), die an die jeweilige Anwendung angepasst werden können.

Die erfindungsgemäße Vorrichtung ist aufgrund der geringen Größe und der Möglichkeit, sie mit Rollen oder Rädern auszugestalten, besonders gut für eine mobile Anwendung geeignet.

Die erfindungsgemäße Vorrichtung ist bei der Débridement-Behandlung mit hoher Effizienz bei vielen verschiedenen Gewebetypen einsetzbar, von Schorf, serösen Krusten, sonstigen Wundbelägen über Sehnen bis hin zu Sehnen und sogar Knochen.

### Die Erfindung im Einzelnen

In einer Ausführungsform der Erfindung entspricht bei der Ultraschallvorrichtung das Gefäß zur Aufnahme der verbrauchten Spül- bzw. Badelösung dem Reaktionsgefäß. Hierdurch resultiert eine besonders kompakte und platzsparende Ausführung, die auch sehr ökonomisch arbeitet, da die verbrauchte Spül- bzw. Badelösung nach Regeneration, bzw. Neuherstellung der NO-haltigen Spül- bzw. Badelösung wieder der Duschvorrichtung zugeführt werden kann.

In einer weiteren Ausführungsform der Erfindung stellen das Reaktionsgefäß und die Badegefäß eigenständige Gefäße dar, die dann bevorzugt über eine Flüssigkeitsleitung miteinander verbunden sind.

Zweckmäßigerweise ist das Reaktionsgefäß als geschlossener Behälter ausgebildet, der jeweils wenigstens einen Zulauf und einen Ablauf für die Spül- bzw. Badelösung aufweist. Ein solcherart geschlossener Behälter erlaubt eine kontrollierte und reproduzierbare Reaktionsführung zur optimierten Herstellung der NO-haltigen Spüllösung. Bei Herstellung des NOs, die mit der Herstellung von toxischen oder unverträglichen Zwischen- oder Endprodukten einhergehen kann, wird dadurch auch die Freisetzung dieser Produkte verhindert. Weiterhin kann bei der Verwendung von UV-Licht im Rahmen der Wirkstoffherstellung (z.B. durch die Photolyse von NO-Donoren) dadurch das Austreten der UV-Strahlung unterbunden werden.

In einer bevorzugten Ausführungsform umfasst die erfindungsgemäße Ultraschallvorrichtung zusätzlich eine oder mehrere Lichtquellen zur Photolyse von NO-Donoren in der Spül- bzw. Badelösung. Diese sind bevorzugt an oder in dem Reaktionsgefäß angeordnet.

In besonders bevorzugter Weise handelt es sich bei diesen Lichtquellen um UV-Lichtquellen.

Zweckmäßigerweise umfasst das Badegefäß zusätzlich eine Auflagefläche zum Aufsetzen oder Auflegen der mindestens einen Körperextremität, die bevorzugt als ebene Platte ausgestaltet ist. Diese Auflage enthält bevorzugterweise mindestens eine Öffnung zum Abfließen der Spül- bzw. Badelösung. Dies hat den Vorteil, dass die auf dieser Auflage aufsetzende oder aufliegende Körperextremität, bevorzugt eine Hand, ein Arm, ein Fuß oder ein Bein nicht direkt in der NO-haltigen Spül- bzw. Badelösung eintaucht, sondern seine NO-Zufuhr ausschließlich durch die Duschvorrichtung erhält, so dass hiermit bei gleichmäßiger NO-Zufuhr eine besonders gute Kontrolle der auf den Körper einwirkenden NO-Exposition resultiert. Zweckmäßigerweise ist diese Auflage als Gitter oder Sieb ausgestaltet, so dass die Spül- bzw. Badelösung schnell und ohne Pfützenbildung abfließen kann. Hierdurch wird verhindert, dass die Körperextremität zu lange mit der NO-haltigen Spül- bzw. Badelösung in Kontakt kommt. Eine Ausgestaltung als Sieb oder Gitter hat den Vorteil, dass bei einer entsprechend engen Maschenweite auch Hautpartikel oder Wundbestandteile festgehalten werden und nicht in das System gelangen. Zweckmäßigerweise stellt die Auflage einen Einwegartikel dar, der nach der Badebehandlung mit den aufgefangenen Partikeln entsorgt werden kann.

In einer weiteren Ausführungsform enthält die Ultraschallvorrichtung in dem Badegefäß unterhalb der Auflage eine oder mehrere Trennabschnitte mit mindestens einer Öffnung zum Abfließen der Spül- bzw. Badelösung, wobei die mindestens eine Öffnung kleiner ist als die die mindestens eine Öffnung der Auflage. Hierdurch können Partikel, die durch die Öffnungen der Auflage hindurchgelassen worden sind, in den darunter befindlichen Trennabschnitten aufgefangen werden und so die Spül- bzw. Badelösung vor dem Eintritt in das Reaktionsgefäß vorgereinigt werden. Bei mehreren Trennabschnitten weisen diese in bevorzugter Weise von oben nach unter eine abnehmende Größe der Öffnungen auf, so dass hierdurch eine stufenweise Filtration der in der Spül- bzw. Badelösung enthaltenden Partikel resultiert.

In einer weiteren Ausführungsform ist das Gefäß zur Aufnahme der verbrauchten Spül- bzw. Badelösung neben, unterhalb oder oberhalb des Badegefäßes angebracht und mit der Behandlungskammer flüssigkeitsführend verbunden. In bevorzugter Weise ist hierbei die Behandlungskammer bevorzugt von dem Gefäß abnehmbar, wie es beispielsweise durch eine Steck-, Bajonett- oder Schraubverbindung realisierbar ist.

In einer bevorzugten Ausführungsform ist das Gefäß zur Aufnahme der verbrauchten Spül- bzw. Badelösung unterhalb des Badegefäßes angebracht und mit dem Badegefäß flüssigkeitsführend verbunden. In bevorzugter Weise ist hierbei das Badegefäß bevorzugt von dem unteren Gefäß abnehmbar, wie es beispielsweise durch eine Steck-, Bajonett- oder Schraubverbindung realisierbar ist.

In einer weiteren Ausführungsform weist die Ultraschallvorrichtung bodenseitig Rollen oder Räder auf, so dass sie leicht auf dem Untergrund bewegbar ist. Hierbei tragen das Reaktionsgefäß und/oder das Badegefäß diese Rollen oder Räder.

### Duschvorrichtung

Erfindungsgemäß umfasst die Ultraschallvorrichtung für das Debridement von Wunden eine Duschvorrichtung.

Eine solche Duschvorrichtung verringert das Risiko der (Re-)kontamination von Wunden durch Mikroben aus der Spül- bzw. Badelösung oder benachbarten Hautarealen, da die kontaminierte Spül- bzw. Badelösung direkt von der Hautstelle abfließt und durch neue, nicht-kontaminierte Spül- bzw. Badelösung ersetzt wird.

Im Gegensatz zu einem Eintauchbad wird die Haut nicht übermäßig aufgeweicht. Da zudem keine Eintauchbehälter gefüllt werden muss, ist diese Art der Anwendung auch schneller und die Behandlung kann direkt nach Generierung der NO-haltigen Spül- bzw. Badelösung beginnen.

Bei instabilen Wirkstoffen, wie beispielsweise NO, erlaubt die Duschanwendung in einfacherer Weise die Herstellung von Spül- bzw. Badelösungen mit konstanter NO-Konzentration.

Eine Duschvorrichtung erlaubt eine flexiblere Anwendung, wobei die Behandlung auf die notwendigen Körperbereiche fokussiert werden kann.

In einer Ausführungsform der Erfindung umfasst die Duschvorrichtung mehrere voneinander beabstandete Duschköpfe, die bevorzugt über eine gemeinsame Flüssigkeitsleitung miteinander verbunden sind.

Erfindungsgemäß umfasst der Duschkopf und/oder das Badegefäß mindestens einen Ultraschallwandler (auch als Schwinger bezeichnet), der elektrische Energie in Ultraschallwellen umwandelt. Entsprechende Ultraschallwandler sind dem Fachmann hinlänglich bekannt und er wird sie entsprechend der Ultraschallfrequenzen, der Intensität und der Dimensionierung aus den bekannten Wandlergeräten auswählen.

Die Ultraschallvorrichtung umfasst in bevorzugter Weise zusätzlich einen Generator zur hochfrequenten Anregung des Ultraschallwandlers.

In einer Ausführungsform ist der mindestens eine Ultraschallwandler im Badegefäß entweder ein Tauchschwinger, ein Plattenschwinger oder ein an der Gefäßwand oder Gefäßboden befestigter Schwinger, so dass im letzteren Fall der Ultraschall dann direkt über Wände und/oder Boden in die Badeflüssigkeit eingeleitet werden kann.

In einer bevorzugten Ausführungsform umfasst der Duschkopf und/oder das Badegefäß genau einen Ultraschallwandler.

Der mindestens eine Ultraschallwandler ist bevorzugterweise dazu ausgerichtet, in einem Bereich von 20 bis 100 kHz und/oder von 1 bis 3 MHz zu arbeiten.

Der Bereich von 20 bis 100kHz wird auch als "Low-Frequency Ultrasound (LFUS) bezeichnet. Der Bereich von 1 bis 3 MHz wird als "High-Frequency Ultrasound (HFUS) bezeichnet. Die Verwendung von HFUS beim Debridement hat den Nachteil, dass es zu einer Erwärmung der angeregten Flüssigkeit kommt, die unter Umständen schädlich für das Gewebe sein kann. Durch eine entsprechende Temperatursteuerung, bzw. Abkühlung der Spüllösung erlaubt die erfindungsgemäße Vorrichtung auch die Verwendung von HFUS zum Debridement.

In einer bevorzugten Ausführungsform ist der mindestens eine Ultraschallwandler dazu eingerichtet, Ultraschall mit einer Schallintensität I von zwischen 0,05 und 1,5 W/cm², bevorzugt von zwischen 0,1 und 1,0 W/cm² und besonders bevorzugt von zwischen 0,1 und 0,8 W/cm² zu erzeugen.

In einer weiteren Ausführungsform ist der mindestens eine Ultraschallwandler zu Erzeugung eines gepulsten Ultraschalls hergerichtet, wobei diese eine Pulsfrequenz von zwischen 1 und 10 Hz aufweist.

In einer Ausführungsform ist der mindestens eine Ultraschallwandler in die Spritzdüse integriert. Hierbei steht der in Schwingung versetzte Teil des Ultraschallwandlers geeigneterweise in Kontakt mit der Spüllösung. Die Spüllösung wird also in direkter Weise mit Ultraschall beaufschlagt.

In einer alternativen Ausführungsform ist der mindestens Ultraschallwandler außerhalb des Sprühkopfs, bspw. also in dem Griff des Duschkopfs integriert, und der schwingende Teil des Ultraschallwandlers steht in Kontakt mit der Düse, welche entsprechend zu Schwingungen angeregt wird und den Ultraschall an die Spüllösung weitergibt. In dieser Ausführung wird die Spüllösung in indirekter Wiese mit Ultraschall beaufschlagt.

In einer weiteren Ausführungsform umfasst bei der Ultraschallvorrichtung auch der Reaktionsbehälter einen Ultraschallwandler. Dieser dient dann dazu, die Spül- bzw. Badelösung herzustellen, insofern feste Bestandteile durch die Ultraschallanregung schneller in der wässrigen Flüssigkeit gelöst werden können.

In einer Ausführungsform wird die in dem Reaktionsgefäß hergestellte NO-haltige Spüllösung durch ein Pumpsystem zu dem Duschkopf transportiert.

Bevorzugterweise ist bei der Ultraschallvorrichtung der Duschkopf durch eine Schlauchleitung mit dem Reaktionsgefäß verbunden und bevorzugt als Handstück ausgestaltet. Unter einem Handstück ist ein Duschkopf zu verstehen, der mit der Hand gehalten werden kann mit der Hand dann an die Wundbereiche herangeführt werden kann.

Der Duschkopf weist zweckmäßigerweise mindestens eine Spritzdüse zur Abgabe der NO-haltigen Spüllösung auf. Es können hierbei auch mehrere Spritzdüsen in einer Spritzdüsenverstelleinrichtung vorgesehen sein, so dass bspw. durch einfaches Drehen der Verstelleinrichtung eine andere Spritzdüse oder eine andere Spritdüsenauswahl zur Anwendung kommen kann.

In einer weiteren Ausführungsform kann der Duschkopf zusätzlich eine oder mehrere Spritzdüsen zur Abgabe einer NO-freien Spüllösung aufweisen. Somit kann man gleichzeitig oder alternierend die Wundbereiche mit der NO-haltigen und der NO-freien Spüllösung behandeln. Hierbei ist es bevorzugt, dass die NO-freie Spüllösung mit Ultraschall beaufschlagt wird, wohingegen die NO-haltige Spüllösung nicht mit Ultraschall beaufschlagt wird. So kann ein für das mechanische Debridement optimierter Sprühstahl neben einem pharmakologisch aktiven, NO-freisetzenden Sprühstrahl verwirklicht werden.

Eine solche Strahloptimierung kann aber auch mit nur einer Spritzdüse erzeugt werden, indem sie dazu ausgelegt und angesteuert ist, abwechselnd einen NO-freien Ultraschallbeaufschlagten Spüllösungsstrahl und eine NO-haltigen schallfreien Strahl zu erzeugen.

In einer Ausführungsform ist die Ultraschallvorrichtung zur Abgabe der NO-haltigen Spüllösung aus dem Duschkopf mit einem Wasserdruck von zwischen 70 kPa und 275 kPa ausgerichtet. Bei einem Wasserdruck in diesem Bereich kommt es zu einer effizienten Debridement ohne dass hier eine Gewebeschädigung eintritt.

In einer weiteren Ausführungsform umfasst der Duschkopf einen austauschbaren Reinigungsaufsatz. Dieser Reinigungsaufsatz sitzt auf der Abgabeseite des Duschkopfs und ist bevorzugt mit einem abrasiv wirkenden Randbereich ausgestattet. Hierdurch kann das durch die pulsierende Flüssigkeit erzielte Debridement mit einem mechanischen, durch den abrasiven Bereich erzielten Debridement kombiniert werden.

Dieser Reinigungsaufsatz kann als mehrfach verwendbares Produkt ausgestattet sein, er stellt aber bevorzugt ein Einwegprodukt dar, dass nach jeder Behandlung durch einen neuen Aufsatz ersetzt wird.

Hierzu ist es bevorzugt, dass der Duschkopf mit einer Befestigungsvorrichtung für den Reinigungsaufsatz versehen ist, so dass dieser einfach und schnell befestigt und auch wieder entfernt, bzw. ausgetauscht werden kann.

Der Reinigungsaufsatz besteht bevorzugt aus Metall oder Kunststoff.

In einer Ausführungsform ist der Duschkopf so ausgestaltet, dass die ultraschallmäßig angeregte Spüllösung kontaktlos an die Wunde abgegeben werden kann. In einer alternativen Ausführungsform wird die Spüllösung über den Reinigungsaufsatz an die Wunde abgegeben. Hierbei steht dann der mit Ultraschall angeregte Reinigungsaufsatz in Kontakt mit der Wunde.

In einer Ausführungsform deckt der Reinigungsaufsatz die Spritzdüse ab und wird so selber zur Verteiler der Spüllösung. In einer alternativen Ausführungsform besitzt der Reinigungsaufsatz in dem Bereich der Spritzdüse eine Aussparung, so dass die Spüllösung von der Spritzdüse direkt an die Wunde abgegeben werden kann.

Hierbei ist es bevorzugt, wenn der Reinigungsaufsatz sich ringförmig um eine zentral angeordnete Spritzdüse erstreckt. Dadurch ist der Flüssigkeitsstrahl ringsum durch den Reinigungsaufsatz abgeschirmt und die Bildung von Aerosolen oder anderen Kontaminationen verringert.

In einer bevorzugten Ausführungsform umfasst der Reinigungsaufsatz eine poröse Reinigungsmatrix. Diese poröse Matrix erlaubt den Durchtritt der Spüllösung zur Wunde. In bevorzugter Weise ist die Reinigungsmatrix ein Polymerschaum, ein Vlies, ein Gewirk oder ein Gewebe.

In einer bevorzugten Ausführungsform ist die Reinigungsmatrix als Einweg-Aufsatz ausgestaltet.

In einer Ausführungsform wird die Reinigungsmatrix mit Ultraschall angeregt. Dadurch kann sie besser mechanisch auf die Wunde einwirken und somit das Debridement positiv unterstützen.

In einer weiteren Ausführungsform ist die Reinigungsmatrix so ausgestaltet, dass sie Wundspülflüssigkeit aufnehmen kann.

In einer Ausführungsform ist die Reinigungsmatrix so ausgestaltet, dass sich mit Flüssigkeit, bevorzugt mit der Spüllösung derart vollsaugen kann, dass eine Flüssigkeitssäule zwischen dem Ultraschallwandler und der Wunde entsteht. Durch die damit erzielte direkte Flüssigkeitsverbindung zwischen dem Ultraschallwandler und der Wunde wird der Ultraschall besser zur Wunde geleitet.

In einer weiteren Ausführungsform ist die Vorrichtung so steuerbar, dass die Ultraschallgenerierung unabhängig von der Zufuhr der Spüllösung gesteuert werden kann. Damit können alterierende "gepulste" Debridementprotokolle etabliert werden. So könnte man zunächst die Wunde mit der NO-haltigen Spüllösung spülen, so dass sich die Reinigungsmatrix mit der NO-haltigen Lösung vollsaugt. Danach würde mit einer NO-freien Lösung der Flüssigkeitskontakt zur NO-haltigen Lösung und somit zur Wunde erzeugt werden und ein Ultraschallimpuls abgegeben werden. Der Vorteil der Reinigungsmatrix besteht hier darin, dass während des Ultraschallimpulses die NO-haltige Spüllösung weiterhin in Kontakt mit der Wunde steht.

In einer weiteren Ausführungsform enthält die Reinigungsmatrix pharmakologisch aktive Substanzen oder Vorstufen von pharmakologischen Substanzen, die bevorzugt durch Ultraschall aktiviert werden können, d.h. dass der Ultraschall die Vorstufe zur Freisetzung der pharmakologisch aktiven Substanz induziert. In einer bevorzugten Ausführungsform handelt es sich bei der Vorstufe um einen Ultraschall-labilen NO-Donor.

So kann die Reinigungsmatrix beispielsweise S-Nitrosogluthathion enthalten. Diese Substanz setzt bei Ultraschallbehandlung das Stickstoffmonoxid frei.

In einer weiteren Ausgestaltung ist an dem Duschkopf eine Umstellvorrichtung vorgesehen, durch die mit dem Duschkopf verschiedene Strahlarten erzeugt werden können, beispielsweise ein normaler Wasserstrahl und ein Brausestrahl.

In einer besonderen Ausführungsform wird durch die Duschvorrichtung ein pulsierender Wasserstrahl erzeugt, der als Massagestrahl die therapeutische Wirkung des Ultraschalls und auch des NOs als gefäßerweiternder Wirkstoff zusätzlich vorteilhaft unterstützt.

In einer besonderen Ausführungsform nutzt der Duschkopf das Prinzip der Venturidüse und ermöglicht die Vermischung einer NO-freien Spüllösung mit der NO-haltigen Spüllösung. In bevorzugter Wiese ist hierbei der in dem Duschkopf integrierten Behälter für die NO-haltige Spül- bzw. Badelösung mit der Wasserzufuhr verbunden. Durch die Verbindung mit der Venturidüse, die sich am Behälter befindet, wird die NO-haltige Spül- bzw. Badelösung aus dem Behälter transportiert und in dieser Mischung an die Duschkopflöcher weitergeleitet. Die Vermischung erfolgt beispielsweise durch Betätigung eines Schalters, der die Verbindung zwischen der Venturidüse und dem Reaktionsbehälter öffnet.

Die Duschvorrichtung ist zweckmäßigerweise so ausgestaltet, dass die Freisetzung von NO aus der Spüllösung in die Luft verhindert wird. Hierzu kann beispielsweise der Duschkopf eine randseitige Luftansaugung aufweisen, so dass das aus dem Sprühstrahl austretende NO sofort abgesaugt wird und entweder im Duschkopf der Spül- bzw. Badelösung wieder zugeführt wird oder aus dem System (z. durch Filtration, Adsorption oder Abbau) entfernt wird.

Dies kann auch durch einen Duschkopf mit zwei unterschiedlichen Austrittsbereichen gewährleistet werden, wobei ein erster, innerer Bereich des Duschkopfs für die NO-haltige Spüllösung vorgesehen ist und ein zweiter ringförmiger Austrittsbereich, der den inneren Bereich umschließt, für eine NO-freie, durch Ultraschall-angeregte Spüllösung vorgesehen ist. Dieser zweite Bereich bildet einen "Mantel" aus NO-freier Spüllösung und sorgt dafür, dass das aus der Spüllösung des ersten mittleren Bereichs austretende NO hierein gelöst wird und nicht in die Umgebung gerät.

In einer weiteren Ausgestaltung der Erfindung ist die Duschvorrichtung nicht als Duschkopf gestaltet, sondern als Schlauch oder Rohr mit Austrittsöffnungen, wobei der Schlauch oder das Rohr bevorzugt als Ring oder als Spirale ausgeformt sind. In einer bevorzugten Ausführungsform ist der Ring oder die Spirale an der Innenwand einer Duschkammer der Duschvorrichtung angebracht und die Austrittslöcher weisen hierbei nach innen.

In einer bevorzugten Ausgestaltung, ist die Duschvorrichtung so ausgestaltet, dass sie auf dem zu behandelnden Körperteil aufgelegt oder befestigt werden kann und so in bevorzugter Weise einen Wasserfilm aufbaut, der an dem Körperteil abläuft. Diese Ausgestaltung hat den Vorteil, dass sie mit besonders geringen Mengen an NO-haltiger Spül- bzw. Badelösung auskommt und durch die Filmbildung (im Gegensatz zu einer Sprühvorrichtung) die Freisetzung von potenziell toxischen Wirkstoffen in die Umwelt besonders gut verhindert. Für diese Ausgestaltung kann der Schlauch oder der (Halb)ring partiell oder vollständig um das zu behandelnde Körperteil herumgeführt werden und beispielsweise durch eine leichte Klemmwirkung an diesem arretiert werden. In einer alternativen Ausgestaltung, kann die Duschvorrichtung auch als Bügel ausgestaltet sein, der in seiner Form an das zu behandelnde Körperteil angepasst ist.

In einer besonderen Ausführungsform ist an dem oben erwähnten Ring, Schlauch- oder Bügel ein Duschvorhang befestigt. Bei der körperseitigen Befestigung dieser Duschvorrichtungen verhindert dieser körpernahe Duschvorhang in zusätzlichem Maße die Freisetzung des Wirkstoffs, der wundseitigen Bakterien und auch der wundseitigen Gewebetrümmer und -bestandteile, die durch das Debridement entfernt werden.

In einer weiteren Ausgestaltung ist die Duschvorrichtung als Drainagestrumpf, Bandage oder Handschuh ausgestaltet und erlaubt so eine gezielte körperseitige NO-Freisetzung.

Des Weiteren kann die Duschvorrichtung mit einer oder mehreren Körperabdeckungen kombiniert werden, so dass nur der zu behandelnde Bereich für die Duschanwendung zugänglich ist. In einer bevorzugten Ausführungsform kann diese Abdeckung eine oder mehrere Aussparungen für den zu behandelnden Körperbereich aufweisen.

In einer weiteren Ausgestaltung ist bei der Duschvorrichtung die Austrittsöffnung schlitzförmig ausgestaltet, so dass die Duschvorrichtung als Schwalldusche fungiert. Gegenüber einem Duschkopf mit vielen einzelnen Wasserstrahlen, führt so eine Schwalldusche zu einer geringeren Freisetzung des NOs in die Umgebung.

Zweckmäßigerweise ist die Duschvorrichtung mit einem Ventilregler ausgestattet, der die Wasserzufuhr regelt.

Darüber hinaus kann die Ultraschallvorrichtung noch einen Druckregler aufweisen, der den Wasserdruck und damit die austretende Menge an Spüllösung reguliert und auch gepulste Spülvorgänge erlaubt.

Erfindungsgemäß ist die wirkstoffhaltige Spüllösung bei den vorab genannten Ausgestaltungen der Duschvorrichtung eine NO-haltige Spüllösung.

In einer Ausführungsform der Erfindung wird durch die Ultraschallvorrichtung ein sprudelndes Bad bereitgestellt. Dies kann durch Einblasen eines Gases erzeugt werden oder durch eine chemische Reaktion, bei der ein Gasbildner wie beispielsweise ein Carbonatsalz durch Ansäuerung der Spül- bzw. Badelösung zur Freisetzung von CO₂-Gas induziert wird.

In einer weiteren Ausführungsform der Erfindung ist die Ultraschallvorrichtung mit einer Vorrichtung versehen, die die Freisetzung von NO in die Umwelt verringert oder gänzlich verhindert. Dies kann eine mechanische Abtrennung sein, die beispielsweise in Form einer Haube oder eine Abdeckfolie das Reaktionsgefäß und/oder die Behandlungskammer abdeckt, wobei sie eine Aussparung für den einzutauchenden Körperteil vorsieht. Alternativ kann es sich um eine Absaugvorrichtung handeln, die das aus der Spül- bzw. Badelösung freigesetzte NO absaugt und entweder der Spül- bzw. Badelösung zuführt oder das NO abbaut bzw. abfiltriert.

In einer bevorzugten Ausführungsform ist das Reaktionsgefäß ein im Wesentlichen geschlossenes, d.h. hermetisch von der Umwelt abgeschottetes System, das lediglich mit dem Behälter zur Aufnahme des zu behandelnden Körperteils in Verbindung steht. Dadurch wird gewährleistet, dass das in dem Reaktionsgefäß erzeugte NO bevorzugt an die Spül- bzw. Badelösung abgegeben wird und nicht in die Umwelt gelangt.

In einer weiteren Ausführungsform umfasst die Badevorrichtung einen NO-Sensor, so dass als Rückkopplung auf den gemessenen NO-Wert das Ausmaß der NO-Generierung flexibel angepasst werden kann.

Dieser NO-Sensor als Messvorrichtung zur Quantifizierung des NO kann in dem Reaktionsgefäß, in dem Badegefäß oder auf der Außenseite des Badegefäßes angebracht sein. In einer besonderen Ausführungsform sorgt die NO-Sensor-assoziierte Steuerung dafür, dass bei Überschreiten eines kritischen NO-Wertes die Badevorrichtung die NO-Generierung gänzlich einstellt.

In einer Ausführungsform der Erfindung wird das Reaktionsgefäß so angesteuert, dass in der Spül- bzw. Badelösung der Gehalt an NO über den Zeitraum der Behandlung konstant gehalten wird.

In einer alternativen Ausführungsform der Erfindung wird das Reaktionsgefäß so angesteuert, dass der Gehalt an NO über den Zeitraum der Behandlung ansteigt oder abfällt.

In einer Ausführungsform der Erfindung ist die Ultraschallvorrichtung so ausgestaltet, dass in diese ein Nachfüllbehälter eingesetzt werden kann, der dann beispielsweise die fertige oder halbfertige Spüllösung enthält. Hierbei kann die fertig formulierte Spüllösung durch den Behälter passend in die Eintauchvorrichtung eingesetzt werden und durch die herstellungsseitig vorgegebene Formulierung wird gewährleistet, dass die therapeutisch optimale Formulierung vorliegt.

In einer bevorzugten Ausführungsform ist die Ultraschallvorrichtung als tragbare Vorrichtung ausgestaltet. Dies erhöht die Patienten-Compliance und ist nicht nur im Klinikalltag, sondern auch im häuslichen Gebrauch von Vorteil.

In einer weiteren Ausführungsform werden die Inhaltstoffe der Spüllösung in bevorzugt vorportionierter Form (sog. Verpackungseinheit) der wässrigen Flüssigkeit hinzugegeben. Da die erfindungsgemäße NO-Generierung auch mit herkömmlichem Leitungswasser möglich ist, kann so der Anwender auf dieses Leitungswasser zurückgreifen und mit den Inhaltsstoffen, die beispielsweise Puffersubstanz, Salze, NOD und Antioxidans umfassen, mischen und so zu einer gebrauchsfertigen Spül- bzw. Badelösung gelangen.

Bei der vorportionierten Form liegen die Inhaltsstoffe bevorzugt in fester Form vor. So können sie als Puder, Pulver, Granulat, Tablette, Filmtablette, Dragee, Weichgelatinekapsel, Hartgelatinekapsel, Oblong, Caplet, Brausetablette oder Pille vorliegen, wobei die Verpackungseinheit zweckmäßigerweise die für jeweils eine Behandlung ausreichenden Menge enthält.

In einer bevorzugten Ausführungsform liegen die Inhaltsstoffe als Brausetablette vor. In dieser Form werden sie schnell gelöst und reichern das Medium zudem mit dem entsprechenden -bevorzugt inerten- Gas (bspw. CO₂) an. Diese Darreichungsform ist zudem im Bereich der Badeanwendungen den Anwendern wohlbekannt und besitzt daher auch eine hohe Compliance.

Alternativ können die Inhaltsstoffe in flüssiger oder halbfester Form vorliegen. Halbfeste Formen umfassen beispielsweise: Suspension, Emulsion, Paste, Creme, Salbe, Gel oder Lotion. Die Vorportionierung als Verpackungseinheit kann beispielsweise durch die Verpackung in Ampullen, Flaschen, Säckchen oder Tuben gewährleistet werden.

In einer weiterhin bevorzugten Ausführungsform ist die Verpackungseinheit so ausgestaltet, dass sie von der Form her eine fehlerfreie Anwendung in der Ultraschallvorrichtung ermöglicht. So ist die Form in bevorzugter Weise als Kartusche ausgestaltet, die nur in einer Orientierung in die Badevorrichtung befestigbar ist. Darüber hinaus kann diese Kartusche mit einem Arretierungsmechanismus ausgestaltet sein, der nur nach korrekter Arretierung in der Ultraschallvorrichtung die Inhaltsstoffe in der gewünschten Weise an das Reaktionsgefäß freigibt. Zweckmäßigerweise kann Ultraschallvorrichtung hierbei mit einem Sensor ausgestattet sein, der eine inkorrekte Orientierung bzw. Arretierung der Kartusche detektiert und dem Anwender anzeigt.

In einem weiterem Aspekt stellt die Offenbarung einen Kit bereit, der eine Verpackungseinheit für eine Behandlung umfasst, wobei diese Verpackungseinheit eine pulverförmige, gelförmige oder flüssige Zusammensetzung enthaltend NOD, Puffersubstanz, Antioxidans und optional ein Lösungsmittel aufweist.

Zur Steuerung der Behandlungsdauer kann die Ultraschallvorrichtung in bevorzugter Weise eine Zeitsteuerungseinheit umfassen, die nach einer fest vorgegebenen oder bevorzugt flexibel programmierbaren Zeit die NO-Generierung abschaltet.

Darüber hinaus kann die Spüllösung einen Farbstoff enthalten, der nach einer bestimmten Zeit eine Farbveränderung erfährt, so dass der Benutzer über das Ende des Behandlungszeitraums informiert ist.

Weiterhin kann die Ultraschallvorrichtung auch eine Vorrichtung zur Messung der Durchblutung umfassen, der anhand des Therapieerfolges eine besonders gute Steuerung der Behandlungsdauer und/oder Behandlungsintensität erlaubt. Dem Fachmann sind zahlreiche Vorrichtungen zur Messung der Durchblutung bekannt. Beispiele hierfür sind Gefäßtachometer, oder der in der WO 97/46853 offenbarte Mikrosensor. Dieser Sensor umfasst einen Indikator-durchlässigen Einsatz, der in einer Öffnung eines Indikator-Behälters, der durch ein Behältnis gebildet ist, angeordnet ist, wodurch der Einsatz einen durchlässigen Wand-Abschnitt des Behälters bildet.

In einer bevorzugen Ausführungsform ist die Vorrichtung zur Messung der Durchblutung, also bevorzugterweise das Gefäßtachometer im Duschkopf integriert.

Als Surrogatparameter für die Hautdurchblutung können weitere vaskulär bedingte Messparameter wie die Rötung der Haut oder die Hauttemperatur dienen, für die entsprechende Messmethoden und -geräte aus dem Stand der Technik bekannt sind.

In einer bevorzugten Ausführungsform ist die Ultraschallvorrichtung mit einer UV-Strahlungsquelle versehen, deren UV-Strahlung durch photolytischen Zerfall das NO direkt in der Spüllösung generiert. Dies hat den Vorteil, dass die Spül- bzw. Badelösung in einem abgeschlossenen Kompartiment vorliegen kann und zudem die NO-Generierung in kontrollierter und reproduzierbarer Weise ablaufen kann.

Bevorzugterweise wird zur NO-Generierung die Spül- bzw. Badelösung in der Ultraschallvorrichtung in einem flachen Reaktionsgefäß von der Strahlungsquelle angestrahlt.

So ist für die photolytische Spaltung ein Reaktionsgefäß mit einer Schichtdicke von zwischen 1 und 20 mm, bevorzugt von zwischen 2.5 und 10 mm und besonders bevorzugt von zwischen 5 und 7 bis 8 mm geeignet. Es zeigte sich, dass eine entsprechend dimensionierten Schichtdicke durch optimale Ausnutzung der UV-Strahlung zu einer hohen Ausbeute an NO führt.

Zweckmäßigerweise ist das Material des Reaktionsgefäßes für UV-Strahlung durchlässig. Aufgrund seiner Kenntnis der UV-Durchlässigkeit wird der Fachmann die geeigneten Materialien für das Reaktionsgefäß auswählen. Bei UV-Strahlung im UV_{A}-Bereich (315 bis 380 nm) kann noch herkömmliches Natron-Kalk-Glas verwendet werden, bei höherenergetischer Strahlung bis 290 nm kann Borosilikatglas zum Einsatz kommen, und bei UV-Strahlung unterhalb von 290 nm ist Quarzglas geeignet.

Auch UV-durchlässige Kunststoffe wie Polymethylpenten (PMP), modifiziertes Polymethylmethacrylat (PMMA), modifiziertes Polyvinylbutyral (Trosivol UV+®) können als Material für das Reaktionsgefäß verwendet werden.

In einer bevorzugten Weise ist das Reaktionsgefäß so ausgeformt, dass es mit seiner der Strahlungsquelle zugewandten Fläche einen definierten, gleichbleibenden Abstand aufweist. Bei einer röhrenförmigen Strahlungsquelle ist das Reaktionsgefäß entsprechend als Hohlzylinder ausgeformt, in dessen Zentrum die Röhre positioniert ist. Die Spül- bzw. Badelösung wird hierbei zweckmäßigerweise an einem Ende des Zylinders zugeführt, strömt über die Länge des Zylinders an der UV-Strahlungsquelle vorbei, wobei es sich zunehmend mit NO anreichert und wird am anderen Ende des Zylinders entnommen, um dem Behandlungsbehälter zugeführt zu werden.

Alternativ kann das Reaktionsgefäß auch ein Rohr sein, das als Spirale mit definiertem Innendurchmesser ausgeformt ist, wobei die röhrenförmige UV-Quelle im Zentrum der Spirale angeordnet ist. Diese Anordnung ermöglicht einen graduellen Anstieg der NO-Konzentration, wobei die NO-Ausbeute hier bei gleichbleibender Strahlungsintensität durch die Fließgeschwindigkeit in der Spirale gesteuert werden kann.

In einer alternativen Ausgestaltung ist bei einer flächenförmigen Strahlungsquelle (z.B. durch ein LED-Panel) das Reaktionsgefäß als flacher Kasten ausgeformt. Dieser weist bevorzugt diametral angebrachten Zu- und Abflüsse für die Spül- bzw. Badelösung auf und kann im Inneren auch Trennwände enthalten, die den Fluss der Spül- bzw. Badelösung in geeigneter Weise steuern können.

In einer weiteren Ausführungsform ist das Reaktionsgefäß auf der von der Strahlungsquelle abgewandten Weise mit einer UV-reflektierenden Beschichtung versehen. Damit kann die Strahlungsausbeute zusätzlich erhöht werden, indem das reflektierte UV-Licht erneut die Spül- bzw. Badelösung durchqueren kann und hierbei NO photolytisch generieren kann. Dem Fachmann sind entsprechende UV-reflektierende Schichten wie bspw. Aluminium oder dielektrische Schichten bekannt. In einer alternativen Ausführungsform ist die UV-reflektierende Beschichtung nicht auf dem Reaktionsgefäß selbst, sondern getrennt dazu angebracht, z.B. auf der Innenwand der Badevorrichtung.

Zweckmäßigerweise umfasst die Ultraschallvorrichtung auch eine Pumpvorrichtung als System zum Umwälzen und/oder Pumpen der Spüllösung.

Diese Pumpvorrichtung kann bei der erfindungsgemäßen Ultraschallvorrichtung in verschiedener Weise zum Einsatz kommen. So kann sie dazu dienen, die im Reaktionsgefäß hergestellte NO-haltige Spüllösung zu der Duschvorrichtung zu transportieren. Weiterhin kann die Pumpvorrichtung auch dem Reaktionsgefäß vorgeschaltet sein und für den Transport der extern bereitgestellten Flüssigkeit in das Reaktionsgefäß dienen.

Zudem kann auch die flüssigkeitsführende Verbindung zwischen dem Badegefäß und dem Gefäß oder der Vorrichtung zu Aufnahme der verbrauchten Spül- bzw. Badelösung eine Pumpvorrichtung umfassen. Hierdurch kann die verbrauchte Badeflüssigkeit aus dem Badegefäß abgepumpt werden, um sie beispielsweise der Entsorgung oder der Filterung zuzuführen.

In einer alternativen Ausführungsform der Erfindung fließt die Spül- bzw. Badelösung mittels Gravitation von dem Badegefäß in das Gefäß oder die Vorrichtung zur Aufnahme der verbrauchten Spül- bzw. Badelösung ab. Das Gefäß oder die Vorrichtung zur Aufnahme der verbrauchten Spül- bzw. Badelösung ist hierbei unterhalb des Badegefäßes angeordnet.

Dem Fachmann sind Pumpvorrichtungen aus dem Stand der Technik bekannt und er kann anhand der relevanten Parameter wie Viskosität der Spül- bzw. Badelösung, erforderliche Pumpleistung, Volumen des Reaktionsgefäßes und des Badegefäßes, Spül/Duschleistung des Duschkopfs die passende Vorrichtung auswählen.

Als Pumpvorrichtungen kommen hier beispielsweise in Betracht: Schlauchpumpen, Membranpumpen, Kolbenpumpen, magnetgekoppelte Pumpen und Impellerpumpen.

In einer Ausführungsform I der Erfindung umfasst bei der Ultraschallvorrichtung die flüssigkeitsführende Verbindung zwischen dem Badegefäß und dem Gefäß oder der Vorrichtung zu Aufnahme der verbrauchten Spül- bzw. Badelösung eine Filtervorrichtung und/oder eine Absorptionsvorrichtung zur Aufreinigung der verbrauchten Spül- bzw. Badelösung.

Diese Filtervorrichtung kann je nach Reinigungszweck unterschiedlich gestaltet sein. Durch einen Partikelfilter können so bspw. ungelöste Partikel der Spül- bzw. Badelösung, Schwebstoffe, Haut und Wundpartikel abgefangen werden.

Durch einen Sterilfilter können (unter Umständen pathogene) Mikroorganismen, die gerade im Wundbereich anzutreffen sind, und die durch die Duschbehandlung aus der Wunde gespült werden entfernt werden.

Durch einen NO und oder NO₂-Filter oder eine NO bzw. NO₂-Absorptionsvorrichtung können hier diese Gase aus der Flüssigkeit entzogen werden. Hierzu kann Aktivkohle, Zeolithe oder Polyphenylensulfid-Polymere (wie bspw. "noXon" der Firma Hoechst AG, Frankfurt, BRD) verwendet werden.

In bevorzugter Weise ist der Filter so ausgestaltet, dass er den NO-Donor, der in bevorzugter Weise ein Nitritsalz ist, aus der Spül- bzw. Badelösung entfernt. Eine derart gefilterte Spül- bzw. Badelösung kann dann ohne Probleme als Haushaltsabwasser, also bspw. über den Ausguss entsorgt werden.

In bevorzugter Weise ist der Filter so ausgestaltet, dass er neben den dem NO-Donor auch die schädlichen Stickstoffoxidspezies, die hier vor allem NO und NO2 darstellen, aus der Spül- bzw. Badelösung entfernt.

In einer weiteren Ausführungsform der Erfindung umfasst das Gefäß oder die Vorrichtung zur Aufnahme der verbrauchten Spül- bzw. Badelösung ein superabsorbierendes Material. Hierdurch kann gerade bei einer geringen Menge an Spül- bzw. Badelösung die verbrauchte Spül- bzw. Badelösung gänzlich gebunden werden und damit in einfacher Weise entsorgt werden. Die superabsorbierenden Materialien können in einem Gefäß enthalten sein. Alternativ können sie auch in eine Vorrichtung wie beispielsweise einem textilen Gebilde vorliegen, dann bevorzugt mit flüssigkeitsundurchlässiger Außenhülle, in das dann die verbrauchte Spül- bzw. Badelösung eingeleitet wird.

In einer weiterhin bevorzugten Ausführungsform enthält der Superabsorber Substanzen, die schädliche oder unerwünschte Bestandteile der Spül- bzw. Badelösung wie Stickoxide, NO-Donoren und hier insbesondere Nitrit, oder auch bakteriellen Kontaminationen, binden, abbauen oder inaktivieren.

In einer Ausführungsform der Erfindung stellt die Vorrichtung zur Aufnahme der verbrauchten Spül- bzw. Badelösung eine Flüssigkeitsleitung zur Weiterleitung an eine von der Badevorrichtung getrennte Entsorgungseinheit dar. Damit weist die erfindungsgemäße Spül- bzw. Badelösung kein Gefäß bzw. Vorrichtung zum Sammeln der Spül- bzw. Badelösung auf, sondern die Flüssigkeitsleitung, die dann bevorzugt mit einer Filtervorrichtung und/oder einer Absorptionsvorrichtung ausgestattet ist, dient zum Abführen der verbrauchten Spül- bzw. Badelösung aus der medizinischen Badevorrichtung. Die Spül- bzw. Badelösung kann hierbei in einen externen Tank, z.B. ein Sammelgefäß geleitet werden oder direkt der Entsorgung (Abfluss, Ausguß) zugeführt werden.

Die Badevorrichtung ist in geeigneter Weise mit einer Temperiervorrichtung versehen. Diese erlaubt durch Heizen und/oder Abkühlen eine Einstellung einer ausgewählten Temperatur. Die Temperatur ist einer der Parameter, die die NO-Ausbeute und die Löslichkeit des generierten NO bestimmen. Zudem kann so bei der Badeanwendung eine für die therapeutische Anwendung optimale Badetemperatur eingestellt werden. Dies kann eine für den Anwender angenehme Temperatur zwischen 23°C und 28°C sein, oder eine Temperatur zwischen 10°C und 20°C, die dadurch die Durchblutung der Haut steigert.

Dem Fachmann sind Temperiervorrichtungen aus dem Stand der Technik bekannt und er kann anhand der relevanten Parameter wie Volumen der Flüssigkeit und Aufheiz- und Abkühlgeschwindigkeiten die passende Vorrichtung auswählen.

In einer bevorzugten Ausführungsform ist eine Temperiervorrichtung insbesondere in Kombination mit einer (UV)-Strahlungsquelle erforderlich, da diese zu einem Aufheizen der Spül- bzw. Badelösung führt. Um einer Überhitzung des Mediums entgegenzuwirken, muss hier die Kühlung bei verlängerter bzw. intensiver Bestrahlung aktiv werden.

In einer weiteren Ausführungsform fungiert die elektromagnetische Strahlungsquelle nicht nur im Rahmen der NO-Generierung, sondern auch als Heizquelle einer Temperiervorrichtung.

### NO-Herstellungsverfahren im Rektionsgefäß

Die NO-haltige Lösung der Badevorrichtung wird in bevorzugter Weise in dem Reaktionsgefäß durch Reaktion eines pH-labilen NO-Donors mit einem Protonendonor und anschließender pH-Werterhöhung durch Verdünnung mit Wasser oder einer neutralen oder basischen Pufferlösung erzeugt.

Es zeigte sich in überraschender Weise, dass dieses Verfahren den komplementären Anforderungen eine NO-Freisetzungskinetik gerecht wird. So kann im aziden Milieu sehr schnell eine therapeutisch relevante Konzentration an NO in dem Trägermedium aufgebaut werden, die dann nach pH-Werterhöhung durch die Verdünnung über einen längeren Zeitraum in kontrollierter Weise aufrechterhalten werden kann.

Üblicherweise erschwert die kurze Halbwertszeit des NO den therapeutischen Einsatz. Mit dem bei der Vorrichtung bevorzugt verwendeten Verfahren kann trotz der kurzen Halbwertszeit durch eine Stabilisierung des NOs in dem neutralen oder basischen Trägermedium der NO-Spiegel für eine ausreichende Zeitspanne aufrechterhalten werden.

Durch die Anwesenheit von Antioxidantien erlaubt das Verfahren die Herstellung von NO in einer Reinheit, wie sie für die therapeutische oder kosmetische Anwendung erforderlich ist.

Aus dem Stand der Technik sind zahlreiche pH-labile und photolabile NO-Donoren bekannt, wie beispielsweise Nitritsalze, NONOate oder Nitrosothiole, auf die der Fachmann hier zurückgreifen kann.

Aufgrund der hochkontrollierten Steuerung der Freisetzung kann das Verfahren gerade in der erfindungsgemäßen Ultraschallvorrichtung eingesetzt werden, um ein Ultraschallinduziertes Ausgasen des in der Spüllösung gelösten NOs zu verhindern. Dies ist insbesondere bei NO als hochpotentem bioaktivem Molekül ein entscheidender Vorteil.

In besonders bevorzugter Weise kommt ein NO-Herstellungsverfahren zum Einsatz, dass die folgenden Schritte umfasst:
(a) Bereitstellen einer ersten Reaktionskomponente umfassend einen pH-labilen NO-Donor;
(b) Bereitstellen einer zweiten Reaktionskomponente, die ein wässrige Lösung mit einem pH-Wert zwischen 4.0 und 5.0 ist;
(c) Vermischen der ersten und zweiten Reaktionskomponente unter Bildung einer wässrigen Lösung mit einem pH-Wert zwischen 4.0 und 5.0 und Generierung von NO zur Ausbildung einer NO-haltigen wässrigen Lösung;
(d) Verdünnen der NO-haltigen wässrigen Lösung aus Schritt (c) mit einer wässrigen Verdünnungslösung um mindestens den Faktor 10, so dass der pH-Wert der Mischung aus Schritt (c) um mindestens eine pH-Wertstufe erhöht wird.

Durch das hier vorgestellte Verfahren kann sehr zügig eine hochreine, NO-haltige Spüllösung im therapeutisch relevanten Konzentrationsbereich hergestellt werden.

Zur Durchführung des Verfahrens weist das Reaktionsgefäß, die im Folgenden beschriebenen drei Behälter auf, oder ist zur Aufnahme dieser drei Behälter vorgesehen:
(a) ein erster Behälter zur Aufnahme der ersten Reaktionskomponente, der über eine Öffnung oder Leitung mit einem zweiten Behälter verbunden ist,
(b) ein zweiter Behälter zur Aufnahme der flüssigen zweiten Reaktionskomponente, wobei dieser Behälter darüber hinaus mit über eine weitere Öffnungen oder Leitungen zum Durchlass von Flüssigkeit mit einem dritten Behälter verbunden ist;
(c) ein dritter Behälter zur Aufnahme der Verdünnungsflüssigkeit;
wobei die Leitungen oder Öffnungen zwischen den einzelnen Behältern durch ein Absperrelement verschlossen ist, dass sich öffnen oder entfernen lässt, um die Verbindung zwischen den einzelnen Behältern herzustellen.

Bei dem Verfahren handelt es sich um ein einfaches Verfahren mit größtenteils bekannten Substanzen, so dass es nicht nur kostengünstig sowie auf wenig komplexe Art und Weise durchzuführen ist, sondern auch bei geringer Fehleranfälligkeit einfach in der therapeutischen Anwendung ist.

Die mit dem Herstellungsverfahren betriebenen Badevorrichtungen eröffnen im Hinblick auf die kennzeichnenden Parameter und auf die Materialauswahl weitere Freiheitsräume. Durch die pH-Werterhöhung in den bevorzugterweise neutralen oder basischen Bereich unterbleibt die Neugenerierung von toxischen NO₂-Radikalen. Durch die Anwesenheit des mindestens einen Antioxidans werden NO₂-Radikale und andere bei der NO-Generierung entstehenden Radikale eliminiert, so dass das Trägermedium mit hochreinem NO angereichert ist.

### Spüllösung

Als Spüllösung kann hier jede Flüssigkeit verwendet werden, die in der Lage ist NO aufzunehmen und auch wieder abzugeben. In bevorzugter Weise ist die Spül- bzw. Badelösung eine wässrige Flüssigkeit.

### NO-Donor

PH-labile NO-Vorstufen (NO-Donoren, NOD) sind im Stand der Technik bekannt und dem Fachmann geläufig.

In einer bevorzugten Ausführungsform sind die pH-labilen NO-Donoren ausgewählt aus der Gruppe enthaltend organische Nitrate, anorganische Nitrate, anorganische Nitrite, organische Nitritester wie Alkylnitrite, Schwefel-, Stickstoff- oder Sauerstoff-Nitrosoverbindungen, NO-Metall-Verbindungen und NO-chelatierende Substanzen.

Beispiele für pH-labile NOD umfassen anorganische Nitrite, Alkylnitrite wie Isopentylnitrit, Diazeniumdiolate (z.B. US Patente Nr. 7,105,502; 7,122,529; 6,673,338), trans[RuCl([15]aneN4)NO]²⁺, Nitrosyl-Liganden, 6-Nitrobenzo[a]pyrol, S-Nitroso-Glutathion, S-Nitroso-Thiole, S-Nitroso-N-Acetyl-D-Penicillamin (SNAP), Nitroanilin-Derivate (siehe US 2013/0224083 A1), 2-Methyl-2-Nitrosopropan, Imidazoyl-Derivate, Nitratester, Hydroxylnitrosamin, Hydroxylamin, Hydroxyharnstoff oder Natrium-Nitroprussid.

In bevorzugter Weise ist der pH-labile NO-Donor ein anorganisches Nitritsalz, das zweckmäßigerweise eine pharmakologisch verträgliche Substanz darstellt. Als solche kommen beispielsweise Nitrite von Alkali- oder Erdalkalimetallen zur Anwendung. Beispielhaft sind hier genannt: LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, oder Ra(NO₂)₂ und Kombinationen hiervon.

Besonders bevorzugt ist hierbei als NOD das NaNO₂, das in weiterhin bevorzugter Weise zusammen mit einer Kombination aus Ascorbinsäure und Trolox als Antioxidantien in der Spül- bzw. Badelösung eingesetzt wird.

Die Konzentration der Nitritsalze bezogen auf das Gesamtgewicht der sie enthaltenden Spül- bzw. Badelösung kann hierbei bis zu 20 Gew.-% betragen, bevorzugt zwischen 0,25 und 10 Gew.-%, besonders bevorzugt zwischen 3 und 7,5 Gew.-%.

In einer alternativen Ausgestaltung kann auch ein Nitratsalz verwendet werden, bei denen eine enzymatische Umwandlung in das entsprechende Nitritsalz möglich ist. Bevorzugt sind hierbei Nitrate von Alkali- oder Erdalkalimetallen zur Anwendung. Beispielhaft sind hier genannt: LiNO₃, NaNO₃, KNO₃, RbNO₃, CsNO₃, FrNO₃, Be(NO₃)₃, Mg(NO₃)₃, Ca(NO₃)₃, Sr(NO₃)₃, Ba(NO₃)₃, oder Ra(NO₃)₃. Die Konzentration der Nitratsalze bezogen auf das Gesamtgewicht der sie enthaltenden Spül- bzw. Badelösung kann hierbei bis zu 20 Gew.-% betragen, bevorzugt zwischen 0,25 und 10 Gew.-%, besonders bevorzugt zwischen 3 und 7,5 Gew.-%.

### Antioxidans

Um die bei der NO-Generierung auftretenden mehrfach oxidierten Stickoxide, Sauerstoffradikalanionen oder Hydroxylradikale zu entfernen, ist es notwendig, dass die Spül- bzw. Badelösung mindestens ein Antioxidans umfasst.

Nach Art des chemischen Wirkmechanismus werden Antioxidantien in Radikalfänger oder Reduktionsmittel unterschieden.

Bei Oxidationsreaktionen zwischen organischen Verbindungen treten vielfach kettenartige Radikalübertragungen auf. Hier werden Stoffe mit sterisch behinderten Phenolgruppen wirksam, die im Ablauf dieser Übertragungen reaktionsträge, stabile Radikale bilden, die nicht weiter reagieren, wodurch es zum Abbruch der Reaktionskaskade kommt (Radikalfänger). Zu ihnen zählen natürliche Stoffe wie die Tocopherole und synthetische wie Butylhydroxyanisol (BHA), Butylhydroxytoluol (BHT) und die Gallate. Sie sind insbesondere bei unpolaren Flüssigkeiten als Spül- bzw. Badelösung anzuwenden.

Des Weiteren können auch Reduktionsmittel mit einem sehr niedrigen Standard-RedoxPotential von weniger als + 0,4 V (bei pH 7,0 und 25°C) eingesetzt werden. Typische Vertreter sind etwa Ascorbinsäure (-0,04 V bei pH 7 und 25°C), Salze der Schwefligen Säure (+0,12 V bei pH 7 und 25 °C) und bestimmte organische schwefelhaltige Verbindungen (z. B. Glutathion, Cystein, Thiomilchsäure), die vorwiegend in wässrigen Spül- bzw. Badelösungen als Trägermedien eingesetzt werden können.

In einer bevorzugten Ausführungsform ist das mindestens eine Antioxidans in der Lage das als NO-Donor im sauren Milieu vorliegende HNO₂ zu NO zu reduzieren. Hierzu muss das Antioxidans als Reduktionsmittel ein Standard-Redoxpotential von weniger als +1,0362 Volt, bevorzugt von weniger als + 0,5 Volt, besonders bevorzugt von weniger als + 0,2 Volt und insbesondere bevorzugt von weniger als 0 Volt aufweisen.

Das mindestens eine Antioxidans ist zweckmäßigerweise in der Lage das schädliche NO₂-Radikal zum NO₂⁻-Anion zu reduzieren. Für eine effektive Eliminierung des NO₂ Radikals sollte das mindestens eine Antioxidans bevorzugt eine bimolekulare Reaktionskonstante k aufweisen, die größer als 1,0 x 10⁶ M⁻¹s⁻¹ und bevorzugt größer als 1,0 x 10⁷ M⁻¹s⁻¹ ist. Geeignete Antioxidantien mit den dazugehörigen Reaktionskonstanten sind in Kirsch et al., 2002 (Biol. Chem 383; 389 -399, s. Tabelle 1) offenbart. Beispielhaft seien hier genannt: Captoprilthiolat, Kaffeesäure, Sinapinsäure, Ferulasäure, Lycopen, Zeaxanthin, Lutein, Astaxanthin, Canthaxanthin, Arachidonat, Gly-Tyr-Dipeptid, Tyrosin, Purine und Pyrimidine wie die Nucleobasen Adenin, Guanin, Cytosin, Thymin, Uracil und die entsprechenden Derivate und Analoga hiervon inklusive der sie enthaltenden Nucleoside und Nucleotide.

In einer weiteren Ausführungsform enthält die verwendete Spül- bzw. Badelösung, die in bevorzugterweise eine wässrige Flüssigkeit ist, neben dem Antioxidans auch einen Antioxidationssynergisten. Synergisten unterstützen die Wirkung von Antioxidantien, indem sie beispielsweise verbrauchte Antioxidantien wieder regenerieren (sog. "Redox cycling"). Durch Komplexierung von Metallspuren (Natrium-EDTA) oder Schaffung eines oxidationshemmenden pH-Wertes können Synergisten die antioxidative Wirkung eines Radikalfängers oder Reduktionsmittels verstärken. Typische Beispiele für Antioxidantionssysnergisten sind EDTA, 1-Hydroxyethan-1.1-diphosphonsäure, Citronensäure, Fumarsäure, Harnsäure und 2-(Hydroxymethyl)-1,4-benzyldiol.

Bei dem Herstellungsverfahren wird besonders bevorzugt das Ascorbat oder die Ascorbinsäure als Antioxidans eingesetzt.

Dem Fachmann sind zahlreiche Antioxidantien bekannt, welche in der Lage sind, mehrfach oxidierte Stickoxide, Sauerstoffradikalanionen, Hydroxylradikale oder aquatisierte Elektronen abzubauen oder zu neutralisieren. Diese wird er entsprechend der jeweiligen Zusammensetzung der Spül- bzw. Badelösung auswählen.

Für apolare Spül- bzw. Badelösungen eignen sich beispielsweise Antioxidantien wie Tocopherole, Tocotrienole, Tocomonoenole, Irganox®, Irgafos®, Butylhydroxyanisol (BHA) und Butylhydroxytoluol (BHT).

Für polare Spül- bzw. Badelösungen, wie bspw. wässrige Flüssigkeiten eignen sich wasserlösliche Vitamin E-Derivate wie Trolox oder alpha-AMG, organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure, Ferulasäure, Sinapinsäure oder Kaffeesäure, oder Hydroxybenzoesäuren wie Gallussäure, Procatechusäure, Syringasäure oder Vanillinsäure.

Andere bevorzugte Antioxidantien umfassen polyphenolische Verbindungen wie Anthocyane, Flavonoide und Phytoöstrogene.

In einer bevorzugten Ausführungsform ist das mindestens eine Antioxidans aus Schritt (a) oder (b) ein Gemisch aus einem Vertreter der Reduktongruppe und einem Vertreter der 6-Hydroxy-Chromangruppe oder der Thiole. Es hat sich herausgestellt, dass eine solche Antioxidantien-Kombination die bei der Reaktion entstehenden schädlichen Radikale besonders wirksam eliminieren kann, ohne dass die NO-Bildung hierdurch beeinträchtigt wird.

In einer Ausführungsform enthält die Spül- bzw. Badelösung und insbesondere in ihrer Ausgestaltung als wässrige Flüssigkeit zusätzlich einen oder mehrere der folgenden Stoffe: Katalysatoren, Detergentien, Puffersubstanzen, Chromophore, Substanzen, die das Prodrug stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung der Spül- bzw. Badelösung zur topischen Anwendung physiologische verträgliche und/oder dermatologisch verträgliche Stoffe und Stoffgemische zur Anwendung kommen.

### Pharmakologische aktive Substanzen

In einer Ausführungsform enthält die Spül- bzw. Badelösung und insbesondere in ihrer Ausgestaltung als wässrige Flüssigkeit eine oder mehrere pharmakologisch aktive Substanzen. Diese können die pharmakologische Wirkung des NOs oder die Ultraschallwirkung unterstützen oder unabhängig von dem NO in einer für die entsprechende Erkrankung therapeutisch relevanten Weise wirken. Beachtlicherweise kann eine durch Ultraschall-angeregte Spüllösung die Aufnahme von Substanzen in die Haut erhöht werden.

In einer Ausführungsform enthält die Spül- bzw. Badelösung und insbesondere in ihrer Ausgestaltung als wässrige Flüssigkeit eine oder mehrere der folgenden pharmakologisch aktiven Substanzen: Entzündungshemmer wie bspw. nichtsteroidale Antirheumatika (NSAIDs) oder Corticoide, Immunsuppressiva, Antibiotika, Antikoagulantien, Antithrombotika, antivirale Agenzien, Antimykotika, Lokalanästhetika und Analgetika.

In einer Ausführungsform enthält die Spüllösung ein NO-generierendes Substrat für die endogenen NO-Synthasen. Es gibt Hinweise, dass eine Ultraschallbehandlung endogene NO-Synthasen aktivieren kann (Altland et al., 2004, Journal Thrombosis Haemostasis, 2 (4): 637-643). Entsprechend würde eine Zuführung von NOS-Substraten zusätzlich die NO-Konzentration im Wundgewebe erhöhen.

### Lichtquelle

In einer Ausführungsform umfasst das Reaktionsgefäß oder eine das Reaktiosngefäß enthaltende Reaktionseinheit eine Lichtquelle, die beispielsweise zur Sterilisierung der Spüllösung oder zur Erzeugung von NO aus einem photolytischen NO-Donor verwendet werde werden kann.

Eine Lichtquelle erzeugt dabei eine elektromagnetische Strahlung, die das Spektrum des sichtbaren Lichts, des Infrarotlichts und insbesondere die UV-Strahlung beinhaltet. Die UV-Strahlung umfasst hierbei sowohl die UV_{A} als auch die UV_{B}-Strahlung.

In bevorzugter Weise strahlt die Lichtquelle UV_{A}-Strahlung mit Wellenlängen von beispielsweise 320 bis 400 nm ab.

Bei der Ultraschallvorrichtung kann die Lichtquelle im Reaktionsgefäß, an dem Reaktionsgefäß (z.B. in der Gefäßwand integriert) oder außerhalb des Reaktionsgefäßes angebracht sein kann. Wichtig ist, dass die Lichtdurchflutung der Spüllösung mitsamt den das Stickstoffmonoxid freisetzenden Reaktionssubstanzen im Sinne eines induzierten Stoffzerfalls bzw. einer Freisetzung von Stickstoffmonoxid maximal ist. Die Quelle der elektromagnetischen Strahlung kann dabei eine mit entsprechenden Fluorochromen beschichtete Glüh- oder Gasentladungslampe (niedrigdruck- oder hochdruckentladend), Licht-emittierende Diode (LED), organische Licht-emittierende Diode (OLED), LASER oder jede andere elektromagnetische Strahlungsquelle sein, die in der Lage ist, aus entsprechenden chemischen Vorstufen bzw. Substraten NO zu generieren. Vorzugsweise sollte bei einer photolytischen Spaltung daher das Reaktionsgefäß zumindest im Bestrahlungsbereich aus einem Material aufgebaut sein, das für die Wellenlängen, die für die NO-Freisetzung relevant sind, durchlässig ist.

### Therapeutische oder kosmetische Verwendung

Die Ultraschallvorrichtung wird zum Debridement von Wunden verwendet, wobei die zu behandelnde Wunde von der NO-haltigen Spüllösung benetzt, ausgespült oder ausgespritzt wird.

In einer bevorzugten Ausführungsform ist die mit der erfindungsgemäßen Vorrichtung behandelte Wunde ausgewählt aus akuten Wunden, chronischen diabetischneuropathischen Ulcus, Ulcus cruris, Dekubituswunden; primär heilende Wunden, sekundär heilende infizierte Wunden und Brandwunden.

In besonders bevorzugter Weise ist die Ultraschallvorrichtung für das Debridement von chronischen Wunden der unteren Extremitäten von Diabetikern geeignet.

### DEFINITIONEN

Im Kontext der vorliegenden Erfindung ist Debridement definiert als das medizinische Vorgehen zur Entfernung von infiziertem, geschädigtem oder abgestorbenem (nekrotischem) Gewebe aus Geschwüren, Verbrennungs- und anderen Wunden oder bei Organzerfall. Das durch Debridement entfernte Material umfasst beispielsweise, nekrotisches Material, fester und viskoser Schorf, seröse Krusten, abgestorbenes und infiziertes Gewebe, Hyperkeratose, Abschilferungen, Eiter, Hämatome, Fremdkörper, Detritus, Knochensplitter und Wundbeläge sonstiger Art. Zur weitergehenden Begriffsklärung mit den Indikationen und Zielen sei auf das Konsenspapier "Debridement" der Euopean Wound Management Association (EWMA) verwiesen (Wound Management, Supplement 3/2013).

Als Wirkstoff wird im Rahmen der Erfindung eine pharmakologisch aktive Substanz verstanden - im Gegensatz zu den Hilfsstoffen. Der Wirkstoff ist also derjenige Bestandteil der Spül- bzw. Badelösung, der eventuell im Zusammenspiel mit den Hilfsstoffen, für die Wirksamkeit der Spül- bzw. Badelösung verantwortlich ist.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

### FIGURENLEGENDEN

Die Erfindung wird nachfolgend anhand der Figuren näher erläutert ohne die Erfindung auf dieses zu beschränken. Es zeigen:

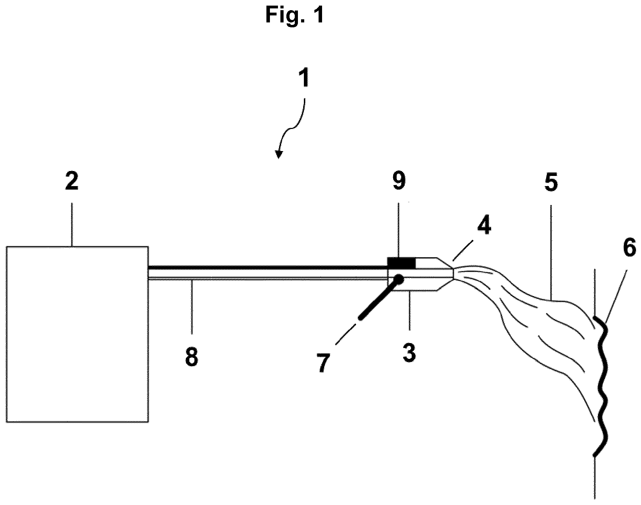

- Fig. 1:: eine schematische Darstellung einer erfindungsgemäßen Ultraschallvorrichtung mit einer Schlauchleitung (8), über die die NO-haltige Spüllösung (5) an den Duschkopf geleitet wird. Der Duschkopf weist einen Ventilregler (7) und einen Ultraschallwandler (9) auf, der die Ultraschallenergie auf die durch die Spritzdüse (4) abgegebene NO-haltige Spüllösung überträgt. Diese Spüllösung überträgt dann ihrerseits die Ultraschallenergie auf die behandelte Wunde (6).

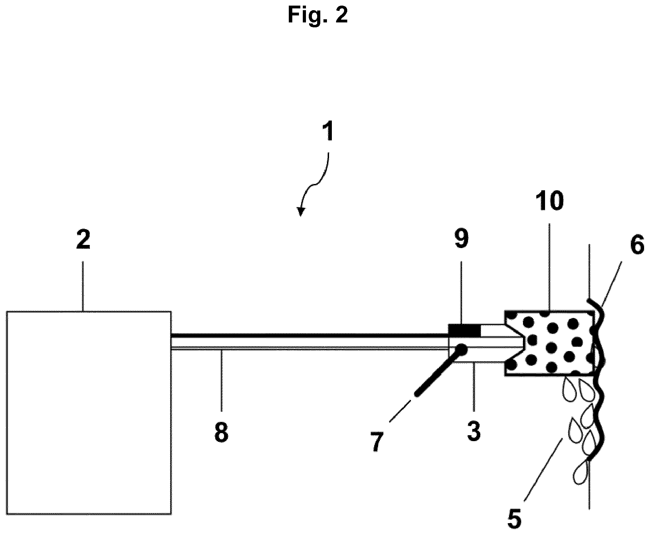

- Fig. 2:: eine schematische Darstellung einer erfindungsgemäßen Ultraschallvorrichtung gemäß Figur 1, wobei der Duschkopf auf der Vorderseite einen Reinigungsaufsatz (10) trägt, der aus einer porösen Reinigungsmatrix zur Aufnahme der NO-haltigen Spüllösung besteht.

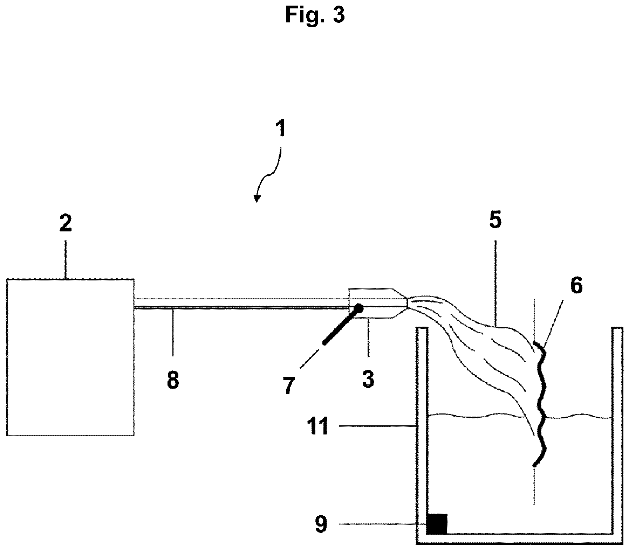

- Fig. 3:: eine schematische Darstellung einer erfindungsgemäßen Ultraschallvorrichtung mit einer Schlauchleitung (8), über die die NO-haltige Spüllösung (5) an den Duschkopf geleitet wird. Der Duschkopf weist einen Ventilregler (7) und einen Spritzdüse (4) zur Abgabe der NO-haltigen Spüllösung auf. Diese Spüllösung sammelt sich in dem Badegefäß (11) als Badelösung an und wird durch den im Badegefäß integrierten Ultraschallwandler (9) ultraschallmäßig angeregt.

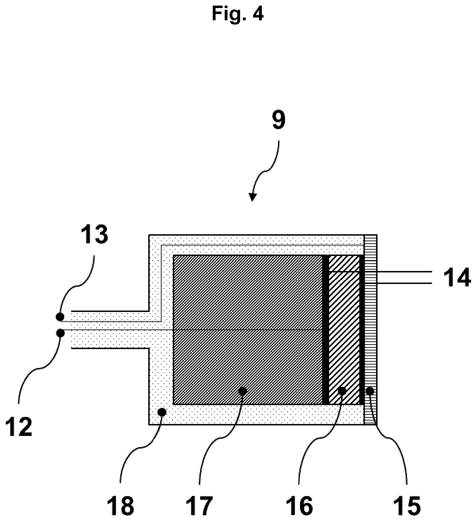

- Fig. 4:: eine schematische Darstellung eines Ultraschallwandlers 9 im Querschnitt mit den Elektroden 14 und den entsprechenden Anschlüssen für die Erdung 13, bzw. den Wechselstrom 12, einem von den Elektroden umfassten Piezokristall 16, einem Dämpfer 17 und einer Masse 18. Der Ultraschallwandler ist frontseitig mit einer Abgleichschicht (sog. "matching layer") ausgestattet, der eine verlustarme Übertragung des Ultraschalls auf die Flüssigkeit ermöglicht.

### BEZUGSZEICHEN

- 1: Ultraschallvorrichtung
- 2: Reaktionsgefäß
- 3: Duschkopf
- 4: Spritzdüse
- 5: NO-haltige Spüllösung
- 6: Wunde
- 7: Ventilregler
- 8: Schlauchleitung
- 9: Ultraschallwandler
- 10: Reinigungsaufsatz
- 11: Badegefäß
- 12: Anschluß für Wechselstrom
- 13: Erdungsanschluß
- 14: Elektroden
- 15: Abgleichschicht ("matching layer")
- 16: Piezokristall
- 17: Dämpfer
- 18: Masse-Gehäuse

## Patentansprüche

1. Ultraschallvorrichtung (1) zur Abgabe einer Stickstoffmonoxid (NO)-haltigen Spüllösung (5) zur Verwendung bei dem Debridement von Wunden umfassend:
ein Reaktionsgefäß (2) zur Herstellung der NO-haltigen Spüllösung (5);
eine Duschvorrichtung mit einem Duschkopf (3) zur Abgabe der NO-haltigen Spüllösung (5); und/oder
ein Badegefäß (11), das zur Aufnahme des zu behandelnden Körperteils und der NO-haltigen Spüllösung (5) als Badelösung vorgesehen ist;
wobei der Duschkopf (3) und/oder das Badegefäß (11) einen Ultraschallwandler (9) zur Ultraschallanregung der NO-haltigen Spül- oder Badelösung (5) und zur Ultraschallanregung tiefer Wundbereiche der damit behandelten Wunden umfassen.

2. Ultraschallvorrichtung (1) gemäß Anspruch 1, **dadurch gekennzeichnet, dass** die in dem Reaktionsgefäß (2) hergestellte NO-haltige Spüllösung (5) durch ein Pumpsystem zu dem Duschkopf (3) transportiert wird.

3. Ultraschallvorrichtung (1) gemäß Anspruch 1 oder 2, **dadurch gekennzeichnet, dass** der Duschkopf (3) durch eine Schlauchleitung (8) mit dem Reaktionsgefäß (2) verbunden ist und bevorzugt als Handstück ausgestaltet ist.

4. Ultraschallvorrichtung (1) gemäß einem der Ansprüche 1 bis 3, **dadurch gekennzeichnet, dass** der Duschkopf (3) eine oder mehrere Spritzdüsen (4) zur Abgabe der NO-haltigen Spüllösung (5) aufweist.

5. Ultraschallvorrichtung (1) gemäß einem der vorangehenden Ansprüche, **dadurch gekennzeichnet, dass** der Duschkopf (3) zusätzlich eine oder mehrere Spritzdüsen zur Abgabe einer NO-freien Spüllösung aufweist, wobei die NO-freie Spüllösung bevorzugt mit Ultraschall beaufschlagt werden kann.

6. Ultraschallvorrichtung (1) gemäß einem der vorangehenden Ansprüche, **dadurch gekennzeichnet, dass** die Vorrichtung (1) zur Abgabe der NO-haltigen Spüllösung aus dem Duschkopf mit einem Wasserdruck von zwischen 70 kPa und 275 kPa ausgerichtet ist.

7. Ultraschallvorrichtung (1) gemäß einem der vorangehenden Ansprüche, **dadurch gekennzeichnet, dass** der Duschkopf (3) einen austauschbaren Reinigungsaufsatz (10) umfasst, der bevorzugt einen abrasiv wirkenden Randbereich aufweist.

8. Ultraschallvorrichtung (1) gemäß Anspruch 7, **dadurch gekennzeichnet, dass** der Reinigungsaufsatz (10) eine poröse Reinigungsmatrix umfasst, die aus einem Material besteht, das ausgewählt ist aus der Gruppe enthaltend Polymerschaum, Vlies, Gewirk oder Gewebe.

9. Ultraschallvorrichtung (1) gemäß einem der vorangehenden Ansprüche, **dadurch gekennzeichnet, dass** der Ultraschallwandler (9) für die Generierung von Ultraschall mit einer Frequenz in einem Bereich von 20 bis 100 kHz und/oder von 1 bis 3 MHz geeignet ist.

10. Ultraschallvorrichtung (1) gemäß einem der vorangehenden Ansprüche, **dadurch gekennzeichnet, dass** der Ultraschallwandler (9) Ultraschall mit einer Schallintensität I von zwischen 0,05 und 1,5 W/cm², bevorzugt von zwischen 0,1 und 1,0 W/cm² und besonders bevorzugt von zwischen 0,1 und 0,8 W/cm² erzeugen kann.

11. Ultraschallvorrichtung (1) gemäß einem der vorangehenden Ansprüche, **dadurch gekennzeichnet, dass** der Ultraschallwandler (9) einen gepulsten Ultraschall mit einer Pulsfrequenz von zwischen 1 und 10 Hz erzeugen kann.

12. Ultraschallvorrichtung (1) gemäß einem der vorangehenden Ansprüche, **dadurch gekennzeichnet, dass** der Ultraschallwandler (9) im Badegefäß (11) entweder ein Tauchschwinger, ein Plattenschwinger oder ein an der Gefäßwand oder Gefäßboden befestigter Schwinger ist, so dass der Ultraschall dann direkt über Wände und/oder Boden in die Badeflüssigkeit eingeleitet wird.

13. Ultraschallvorrichtung (1) gemäß einem der vorangehenden Ansprüche, **dadurch gekennzeichnet, dass** sie zusätzlich einen Generator zur hochfrequenten Anregung des Ultraschallwandlers umfasst.

14. Ultraschallvorrichtung (1) gemäß einem der vorangehenden Ansprüche, **dadurch gekennzeichnet, dass** sie eine tragbare Vorrichtung ist.

15. Ultraschallvorrichtung (1) gemäß einem der vorangehenden Ansprüche zur Verwendung bei der Débridement-Behandlung von chronischen Wunden, wobei die chronischen Wunden bevorzugt chronische Wunden der unteren Extremitäten von Diabetikern sind.

## Claims

1. Ultrasonic device (1) for delivering a flushing solution (5) containing nitrogen monoxide (NO) for use in the debridement of wounds, comprising:
a reaction vessel (2) for producing the flushing solution (5) containing NO;
a shower device with a shower head (3) for delivering the flushing solution (5) containing NO; and/or
a bathing vessel (11), which is provided to receive the body part to be treated and the flushing solution (5) containing NO as a bathing solution;
wherein the shower head (3) and/or the bathing vessel (11) comprise an ultrasonic transducer (9) for ultrasonic excitation of the flushing or bathing solution (5) containing NO and for ultrasonic excitation of deep wound areas of the wounds treated thereby.

2. Ultrasonic device (1) according to claim 1, **characterised in that** the flushing solution (5) containing NO and produced in the reaction vessel (2) is transported to the shower head (3) by a pump system.

3. Ultrasonic device (1) according to claim 1 or 2, **characterised in that** the shower head (3) is connected by a hose line (8) to the reaction vessel (2) and is designed preferably as a handpiece.

4. Ultrasonic device (1) according to one of claims 1 to 3, **characterised in that** the shower head (3) has one or more spray nozzles (4) for delivering the flushing solution (5) containing NO.

5. Ultrasonic device (1) according to any one of the preceding claims, **characterised in that** the shower head (3) additionally has one or more spray nozzles for delivering a NO-free flushing solution, wherein the NO-free flushing solution can preferably be acted upon by ultrasound.

6. Ultrasonic device (1) according to any one of the preceding claims, **characterised in that** the device (1) for delivering the flushing solution (5) containing NO from the shower head is configured with a water pressure of between 70 kPa and 275 kPa.

7. Ultrasonic device (1) according to any one of the preceding claims, **characterised in that** the shower head (3) comprises an interchangeable cleaning attachment (10), which preferably has an abrasively operating edge region.

8. Ultrasonic device (1) according to claim 7, **characterised in that** the cleaning attachment (10) comprises a porous cleaning matrix, which consists of a material selected from the group containing polymer foam, nonwoven material, knitted fabric or woven fabric.

9. Ultrasonic device (1) according to any one of the preceding claims, **characterised in that** the ultrasonic transducer (9) is suitable for generating ultrasound at a frequency in a range from 20 to 100 kHz and/or from 1 to 3 MHz.

10. Ultrasonic device (1) according to any one of the preceding claims, **characterised in that** the ultrasonic transducer (9) can generate ultrasound with a sound intensity I of between 0.05 and 1.5 W/cm², preferably of between 0.1 and 1.0 W/cm² and especially preferably of between 0.1 and 0.8 W/cm².

11. Ultrasonic device (1) according to any one of the preceding claims, **characterised in that** the ultrasonic transducer (9) can generate a pulsed ultrasound with a pulse frequency of between 1 and 10 Hz.

12. Ultrasonic device (1) according to any one of the preceding claims, **characterised in that** the ultrasonic transducer (9) in the bathing vessel (11) is either a submersible transducer, a plate transducer or a transducer attached to the vessel wall or vessel floor, so that the ultrasound can be introduced into the bathing liquid directly via walls and/or floor.

13. Ultrasonic device (1) according to any one of the preceding claims, **characterised in that** it additionally comprises a generator for the high-frequency excitation of the ultrasonic transducer.

14. Ultrasonic device (1) according to any one of the preceding claims, **characterised in that** it is a portable device.

15. Ultrasonic device (1) according to any one of the preceding claims for use in the debridement treatment of chronic wounds, wherein the chronic wounds are preferably chronic wounds of the lower extremities of diabetics.

## Revendications

1. Dispositif à ultrasons (1) servant à distribuer une solution de rinçage (5) contenant du monoxyde d'azote (NO) destinée à être utilisée lors du débridement de plaies, comprenant :
un récipient de réaction (2) servant à fabriquer la solution de rinçage (5) contenant du NO ;
un dispositif de douche avec une pomme de douche (3) servant à distribuer la solution de rinçage (5) contenant du NO ; et/ou
un récipient de bain (11), qui est prévu pour recevoir la partie du corps à traiter et la solution de rinçage (5) contenant du NO en tant que solution de bain ;
dans lequel la pomme de douche (3) et/ou le récipient de bain (11) comprennent un convertisseur à ultrasons (9) servant à l'excitation ultrasonore de la solution de rinçage ou de bain (5) contenant du NO et servant à l'excitation ultrasonore de zones de plaie profondes des plaies ainsi traitées.

2. Dispositif à ultrasons (1) selon la revendication 1, **caractérisé en ce que** la solution de rinçage (5) contenant du NO fabriquée dans le récipient de réaction (2) est transportée par un système de pompage à la pomme de douche (3).

3. Dispositif à ultrasons (1) selon la revendication 1 ou 2, **caractérisé en ce que** la pomme de douche (3) est reliée par un conduit flexible (8) au récipient de réaction (2) et est configurée de manière préférée sous la forme d'une pièce à main.

4. Dispositif à ultrasons (1) selon l'une quelconque des revendications 1 à 3, **caractérisé en ce que** la pomme de couche (3) présente une ou plusieurs buses de pulvérisation (4) servant à distribuer la solution de rinçage (5) contenant du NO.

5. Dispositif à ultrasons (1) selon l'une quelconque des revendications précédentes, **caractérisé en ce que** la pomme de douche (3) présente en supplément une ou plusieurs buses de pulvérisation servant à distribuer une solution de rinçage sans NO, dans lequel la solution de rinçage sans NO peut être soumise de manière préférée à l'action d'ultrasons.

6. Dispositif à ultrasons (1) selon l'une quelconque des revendications précédentes, **caractérisé en ce que** le dispositif (1) est configuré pour distribuer la solution de rinçage contenant du NO provenant de la pomme de douche avec une pression d'eau entre 70 kPa et 275 kPa.

7. Dispositif à ultrasons (1) selon l'une quelconque des revendications précédentes, **caractérisé en ce que** la pomme de douche (3) comprend un embout de nettoyage (10) interchangeable, qui présente de manière préférée une zone de bord à effet abrasive.

8. Dispositif à ultrasons (1) selon la revendication 7, **caractérisé en ce que** l'embout de nettoyage (10) comprend une matrice de nettoyage poreuse, qui consiste en un matériau qui est choisi parmi le groupe contenant du mousse polymère, du non-tissé, du tissu à mailles ou du tissu.

9. Dispositif à ultrasons (1) selon l'une quelconque des revendications précédentes, **caractérisé en ce que** le convertisseur à ultrasons (9) est apte à la génération d'ultrasons avec une fréquence dans une plage de 20 à 100 kHz et/ou de 1 à 3 MHz.

10. Dispositif à ultrasons (1) selon l'une quelconque des revendications précédentes, **caractérisé en ce que** le convertisseur à ultrasons (9) peut générer des ultrasons avec une intensité sonore I entre 0,05 et 1,5 W/cm², de manière préférée entre 0,1 et 1,0 W/cm² et de manière particulièrement préférée entre 0,1 et 0,8 W/cm².

11. Dispositif à ultrasons (1) selon l'une quelconque des revendications précédentes, **caractérisé en ce que** le convertisseur à ultrasons (9) peut générer des ultrasons pulsés avec une fréquence d'impulsion entre 1 et 10 Hz.

12. Dispositif à ultrasons (1) selon l'une quelconque des revendications précédentes, **caractérisé en ce que** le convertisseur à ultrasons (9) dans le récipient de bain (11) est soit un oscillateur immersible, un oscillateur à plaques ou un oscillateur fixé au niveau de la paroi de récipient ou du fond de récipient de sorte que les ultrasons sont introduits alors directement en passant par des parois et/ou fonds dans le liquide de bain.

13. Dispositif à ultrasons (1) selon l'une quelconque des revendications précédentes, **caractérisé en ce qu'**il comprend en supplément un générateur servant à l'excitation à haute fréquence du convertisseur à ultrasons.

14. Dispositif à ultrasons (1) selon l'une quelconque des revendications précédentes, **caractérisé en ce qu'**il est un dispositif portatif.

15. Dispositif à ultrasons (1) selon l'une quelconque des revendications précédentes, pour l'utilisation lors d'un traitement de débridement des plaies chroniques, les plaies chroniques étant de préférence des plaies chroniques des extrémités inférieures.
